# EUROPÄISCHE PATENTANMELDUNG

(11) **EP 2 452 624 A1**
(43) Veröffentlichungstag der Anmeldung: **16.05.2012**
(21) Anmeldenummer: 11185354.5
(22) Anmeldetag: 17.10.2011
(51) Int. Cl.: A61B 5/1473, A61B 5/145, B01D 69/14, B01D 71/06, F16K 99/00, F15C 1/00

(54) **Implantierbarer theranostischer Artikel**

(30) Priorität: 12.11.2010 US 412802 P
(71) Anmelder: BIOTRONIK SE & Co. KG, 12359 Berlin (DE)
(72) Erfinder: Borck, Alexander, 91086 Aurachtal (DE); Bunge, Andreas, 04105 Leipzig (DE); Biela, Sarah, 12053 Berlin (DE)
(74) Vertreter: Lindner-Vogt, Karin L.

(57) **Zusammenfassung**

Die vorliegende Erfindung beschreibt einen theranostischen Artikel, umfassend auf seiner Oberfläche spezifische Erkennungsmarker für Zellen, wobei die Erkennungsmarker ausgewählt sind aus der Gruppe bestehend aus Peptiden, Proteinen, Antikörpern, Antigenen, Aptameren, molekular-geprägten Polymeren und Polynukleotiden.

## Beschreibung

Die Erfindung betrifft einen implantierbaren theranostischen Artikel, umfassend auf seiner Oberfläche spezifische Erkennungsmarker für Zellen, die Verwendung von spezifischen Erkennungsmarkern für Zellen zur Verhinderung von Biofouling und Thrombogenität auf der Oberfläche eines Implantates und ein Verfahren zum Herstellen eines implantierbaren theranostischen Artikels mit einer biofoulingvermindernden oder biofoulingverhinderden Oberfläche.

Ein theranostischer Artikel (hier auch "Artikel" genannt) trägt mindestens eine diagnostische, sensorische und/oder therapeutische Funktion. Dieser Artikel kann auch als theranostisches Implantat (im Folgenden kurz "Implantat" genannt) ausgelegt sein.

Implantate haben insbesondere bei Blutkontakt große Probleme mit der Langzeitstabilität. Typischerweise zeigen sie nach dem Einbringen in den Körper eine unspezifische Proteinadsorption. Diese adsorbierten Proteine verlieren ihre Tertiär- bzw. Quartärstruktur wenigstens teilweise und dienen Zellen als Ankersubstrate für eine Anlagerung. Hierdurch ausgelöst bildet sich eine undefinierte Zellbedeckung und/oder eine extrazelluläre Matrix aus Protein-Fasern (z. B. Kollagen) auf der Oberfläche des Artikels aus. Dieser Vorgang wird allgemein als Biofouling bezeichnet. Außerdem kann es kann zu einer Thrombenbildung an der Oberfläche der Implantate kommen.

Wenn es sich bei dem Artikel um einen Sensor handelt, bedeuten diese Ablagerungen, dass sich für die Analyten eine zeitlich nicht stabile Diffusionsbarriere aufbaut, die direkte Auswirkungen auf das Messergebnis zeigt. Durch die Zellbedeckung bzw. die extrazelluläre Matrix kann es sowohl zu einer Drift wie auch zu einem Delay des Messsignals kommen, so dass der Sensor nur mit einer zeitlichen Verzögerung äußere Veränderungen der Analytenkonzentration detektieren kann. Die Diffusionsbarriere kann so groß werden, dass die Analyten nicht mehr zum Sensor gelangen und hier kein Signal mehr abgeleitet werden kann.

Im Stand der Technik werden verschiedene Ansätze beschrieben, um über Oberflächenmodifikationen des Artikels die vorbeschrieben Vorgänge, die durch die unspezifische Anlagerung von Biomolekülen ausgelöst werden, wie Biofouling und Thrombenbildung zu verhindern (Anti-Biofouling-Beschichtungen). Gleichzeitig oder alternativ wird versucht über eine entsprechende Beschichtung der Oberfläche des Artikeles, die Reaktion des Körpers auf dieses positiv zu beeinflussen.

Um die unspezifischen Proteinanlagerungen zu verhindern werden häufig hydrophile Polymerstrukturen, wie z. B. Polyethylenglycol kovalent an die Oberfläche des Artikels gebunden (PEG-ylierungen).

Alternativ wird über Mikro- und Nanostrukturierung der Oberfläche versucht, das Einwachsverhalten der eingebrachten Artikel zu beeinflussen. Dieser Ansatz ist bisher nur für den subkutanen Einsatz bekannt.

So offenbart Gilligan et al in "Feasibility of Continuous Long-Term Glucose Monitoring from a Subcutaneous Glucose Sensor in Humans", Diabetes Technology & Therapeutics, Volume 6, Number 3, 2004 einen implantierbaren Sensor, dessen Oberflächen über Mikround Nanostrukturierung ein positives Einwachsverhalten bewirken sollen.

Ein vergleichbarer Ansatz findet sich auch in Updike et al "A Subcutaneous Glucose Sensor With Improved Longevity, Dynamic Range, and Stability of Calibration", Diabetes Care, Volume 23, Number 2, February 2000.

Im Regelfall sollen die Artikel über längere Zeit im Körper verbleiben. Dabei reagiert der Körper auf das Einbringen des Artikels als Fremdkörper. In diesem Zusammenhang ist es wichtig, dass die Körperreaktionen, wie z. B. Entzündung oder Einwachsen weder den Nutzen des Artikels konterkarieren, in dem sie den Körper mehr belasten als der Artikel nutzt, noch die Funktion des Artikels wesentlich beeinträchtigen. So sind z. B. implantierbare Sensoren medizinisch nur sinnvoll, wenn Sie über einen längeren Zeitraum, bevorzugt wenigstens 1 Jahr im Körper verbleiben können und über diesen Zeitraum stabile Messwerte liefern. Bislang gibt es keine solchen implantierbaren, langzeitstabilen Sensorsysteme, die Messsignale ausreichender Qualität über den Zeitraum von einem Jahr oder länger liefern. Hierfür ist insbesondere das Biofouling verantwortlich. Dabei können-wie bereits angedeutet - unspezifische Protein- bzw. Zellablagerungen und Thromben aus den Körperflüssigkeiten den Zugang für Analyten zur eigentlichen Sensoreinheit einschränken oder sogar verhindern und damit den Sensor unbrauchbar machen. Auch die zeitliche Veränderung der Zugangsmöglichkeiten für den Analyten zum Sensorbereich durch die dynamischen Ablagerungs- und/oder Wachstumsprozesse an der Sensoroberfläche bewirkt eine unzureichende Signalqualität.

Das sich auf dem Markt befindende semiimplantierbare System von Medtronic (MiniMed Paradigm REAL-Time Revel^{TM}) zur subkutanen Bestimmung der Glukosekonzentration ist dementsprechend nur für einen Gebrauch von maximal 7 Tagen von der FDA zugelassen. Um den Signaldrift durch die oben genannten Effekte und zusätzliche Fehlerquellen zu kompensieren, ist mehrmals täglich eine Kalibration über Blutentnahme erforderlich.

Auf dem Markt findet sich zur Zeit kein implantierbarer oder semiimplantierbare Sensor für andere molekulare Blutbestandteile als Glukose. Sinnvoll zur Überwachung von chronischen Erkrankungen wären eine ganze Reihe von Blutbestandteilen wie z.B. Elektrolyte, Stoffwechselprodukte, Bicarbonat, Kreatinin, Harnstoff, Cystatin C und andere Proteine. In der Praxis werden derzeit regelmäßige Bluttests durch Blutabnahme beim Arzt vorgenommen.

Die Oberflächenmodifikationen zur Verminderung oder Unterbindung des Biofoulings durch Beschichtung mit Polymeren (z. B. PEG-ylierungen) funktionieren im Regelfall im ausreichenden Maße nur für eine kurze Zeit nach der Implantation des Artikels. In Kontakt insbesondere mit Körperflüssigkeiten werden die Polymere verhältnismäßig schnell chemisch modifiziert, was zu einem Verlust der Anti-Biofoulingeigenschaften führen kann. Bei den mit Anti-Biofoulingeigenschaften ausgerüsteten Oberflächen ist zusätzlich die ständige Gefahr vorhanden, dass diese Oberflächen die Gerinnungskaskade auslösen und somit thrombogen wirken. Daher kann es erforderlich sein, dass ein Individuum, das ein entsprechendes Implantat trägt, eine Langzeittherapie mit gerinnungshemmenden Stoffen wie z. B. der Dual Antiplatelettherapie erhält.

Die Modifikation durch Nano- und Mikrostrukturierungen zur Beeinflussung der Einheilung ist relativ unspezifisch. Zwar wird versucht, dem Gewebe und den Zellen bestimmte Topologien auf der Artikeloberfläche zu präsentieren, die ein Einwachsen positiv beeinflussen können, jedoch kann nicht gesteuert werden, welche Zellen sich an der Oberfläche anlagern. Dies ist jedoch insbesondere bei Kontakt des Implantates mit dem Blutstrom von hoher Bedeutung. Im Stand der Technik haben sich die Untersuchungen mit implantierbaren biochemischen Sensoren bisher nur mit dem subkutanen Einsatz beschäftigt, ein Einsatz solcher Sensoren mit nano- und mikrostrukturierten Oberflächen (im dauerhaften) Kontakt mit Blutbahnen ist bislang nicht beschrieben.

Als Beispiel in der Literatur, wo strukturierte Oberflächen eingesetzt werden, um das Einwachsverhalten subkutan zu beeinflussen, können die beiden oben genannten Veröffentlichungen aufgeführt werden. Die in diesen beiden Veröffentlichungen beschriebenen Sensoren werden mit einer Membran versehen, die eine Strukturierung aufweist und das Gewebe zur Ausbildung neuer Blutgefäße (Angiogenese) bringt. So kann wenigstens teilweise eine fibröse Einkapselung verhindert werden. Dies bewirkt, dass die Lebensdauer, also die Dauer, in der der Sensor klinisch verwertbare Messwerte liefert, auf bis zu 5 bis 6 Monate verlängert werden konnte. Jedoch ist bislang unklar, ob die beschriebenen Modifikationen der Oberfläche ebenfalls das Biofouling positiv beeinflussen können und ob ein Einsatz (in dauerhaften) Kontakt mit Blut möglich ist.

Vor diesem Hintergrund war es Aufgabe der vorliegenden Erfindung möglichst lange im Körper hinsichtlich ihrer Funktion stabile Implantate mit einer theranostischen Funktion bereitzustellen, bei denen insbesondere das Biofouling deutlich verringert oder bevorzugt gänzlich verhindert wird. Bevorzugt sollten sich diese Vorteile auch bei Kontakt mit dem Blutstrom zeigen.

Erfindungsgemäß wird diese Aufgabe gelöst durch einen implantierbaren theranostischen Artikel, umfassend auf seiner Oberfläche spezifische Erkennungsmarker für Zellen, wobei die Erkennungsmarker ausgewählt sind aus der Gruppe bestehend aus Peptiden, Proteinen, Antikörpern, Antigenen, Aptameren, molekular-geprägten Polymeren und Polynukleotiden mit n ≥ 1 Monomereinheiten (Ribonukleinsäure - RNA, Desoxyribonukleinsäure - DNA, Peptid-Nukleinsäure - PNA, Locked-Nukleinsäuren - LNA).

Im Sinne der vorliegenden Erfindung bedeutet molekular geprägt die Bereitstellung eines Polymergerüstes mit Erkennungsdomänen.

Durch molekulares Prägen sind mit Informationen versehene Polymere zugänglich. Beispielsweise können Polymere durch radikalische Polymerisation in Gegenwart eines Templates (das sich im günstigsten Fall durch Waschschritte wieder entfernen lässt) mit der nötigen Selektivität versehen werden, um zu ähnlichen Strukturen Affinitäten auszubilden. Ein solches Verfahren ist aus Vaidya, A., Borck, A., Manns, A. & L. Fischer. 2004: "Altering glucose oxidase to oxidase Dgalactose through crosslinking of imprinted protein" (ChemBioChem 5 (1): 132-135) bekannt.

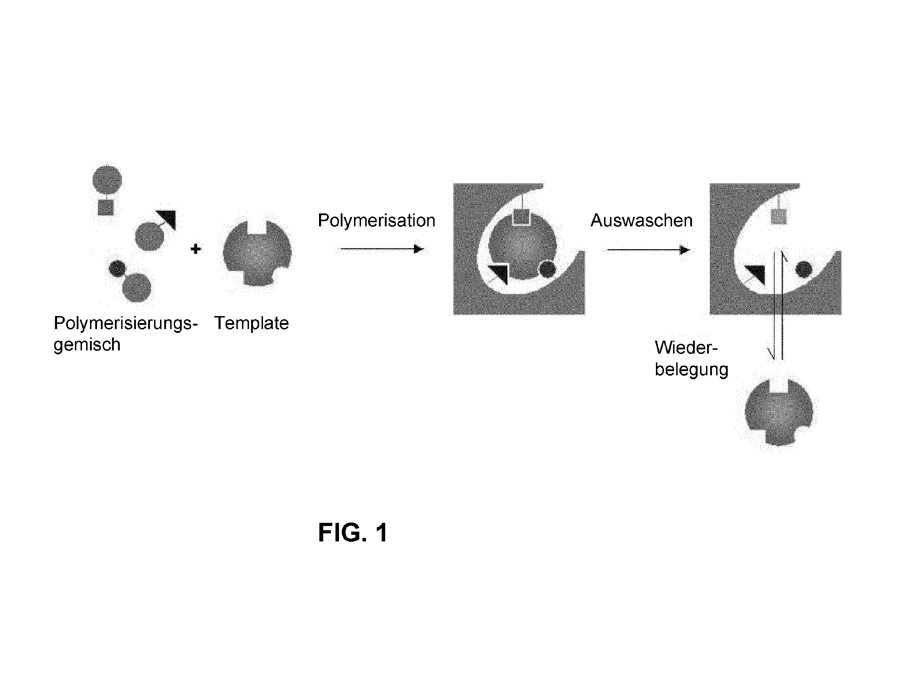

Fig. 1 zeigt eine schematische Darstellung des molekularen Imprintens von Polymeren. Die Art des Epitops bestimmt dabei die Polymerauswahl. Geeignet sind Acrylate und Methacrylate als Grundgerüst. AIBN ist der bevorzugte Radikalstarter. Die Reaktion kann in Lösungsmitteln wie Dioxan, CHCl3 oder THF, aber auch in Substanz durchgeführt werden. Durch Copolymerisate mit Acylacetat (hydrophile Gruppen; können zur Erzeugung von OH-Gruppen gut verseift werden), Acylamin; Acylsäuren oder Styrol (Wechselwirkung mit aromatischen Epitopen) können neben hoher Selektivität auch hohe Bindungsaffinitäten erzeugt werden.

Ein "spezifischer Erkennungsmarker" im Sinne dieser Erfindung ist dabei ein Molekül, das spezifisch für die Anlagerung eines bestimmten Zelltypes ausgebildet ist. Mit anderen Worten, ein "Erkennungsmarker für Zellen" im Sinne dieser Erfindung ist eine Verbindung oder ein Teil einer Verbindung, der von einem, zwei oder drei Zelltypen (bevorzugt einem) spezifisch erkannt wird und eine Bindung der Zellen dieses Typus oder dieser Typen an einer Oberfläche, auf der sich der Erkennungsmarker befindet, bewirken kann. Zellen anderer Typen zeigen dagegen keine solche Reaktion. Im vorliegenden Fall wird bevorzugt die Migration und Proliferation von Endothelzellen (EC) gefördert.

Bevorzugt sind dabei allgemein die Zelltypen, die mit Hilfe von Transmembranproteinen (Integrinen) die Erkennungsmarker auf der Implantatsoberfläche erkennen, ausgewählt aus der Gruppe, der Zellen, die Integrine tragen. Besonders bevorzugt sind dabei Zellen, die zur alfaVBeta3 (αvβ3) Unterfamilie gehören. Neben den schon genannten Endothelzellen besitzen auch Endothelvorläuferzellen die gewünschten Erkennungssequenzen. (Blind et al.: "A novel Drug-Eluting Stent coated with an Integrin-Binding Cyclic Arg-Gly-Asp Peptide Inhibits Neointimal Hyperplasia by Recruiting Endthelial Progenitor Cells"; JA College of Cardiology; Vol. 47, No9, 2006; Garcia A.J.: Get a Grip, integrins in cellbiomaterial interactions"; Biomaterials 26; 7525-7529, 2005)

Die Verwendung von Peptidsequenzen auf Stents ist in WO 2008/143933 A1 beschrieben. Hier soll ebenfalls ein beschleunigtes Einheilen, durch Ausbildung eines Zellrasens, erreicht werden.

Das beschriebene Vorgehen ist für theranostische Anwendungen noch nicht bekannt. Bekannt und Stand der Technik sind Bemühungen die Gewebsbedeckung zu kontrollieren respektive wie durch PEG-ylierungen zu verhindern.

Das Vorsehen bestimmter Erkennungsmarker an der Oberfläche erfindungsgemäßer implantierbarer theranostischer Artikel bewirkt bei der geeigneten Auswahl der Marker abhängig z. B. vom Einsatzort des implantierbaren theranostischen Artikels, dass die Artikeloberfläche im implantierten Zustand eine Grenzfläche präsentiert, auf die der Körper mit einer definierten und nach einem bestimmten Zeitraum sich nicht weiter verändernden dünnen Vernarbung bzw. Verkapselung reagiert. Überraschend ist in diesem Zusammenhang insbesondere, dass nach einer verhältnismäßig kurzen Anwachsphase ein im Prinzip sich nicht weiter veränderndes Gewebe auf den implantierten theranostischen Artikeln entsteht. Durch diese biomimetische Oberfläche wird der Artikel dann nicht mehr vom Körper als fremdes Objekt identifiziert.

Der Kern der Erfindung stellt somit eine neue Herangehensweise dar. Während in der Vergangenheit verstärkt versucht wurde, bioinerte Oberflächen und Nanostrukturierungen für Implantatanwendungen zu schaffen, die mit dem Nachteil der Thrombogenität und mangelnden Spezifität verbunden waren, wird mit der vorliegenden Erfindung erreicht, dass durch die spezifischen Erkennungsmarker für Zellen eine (weitgehend) konstante und biomimetische Oberfläche entsteht. Hierbei ist der Umfang der Zellbedeckung auch durch die Konzentration der spezifischen Erkennungsmarker für Zellen auf der Oberfläche des implantierbaren theranostischen Artikels steuerbar. Im Prinzip entsteht aber eine verhältnismäßig geringe Zellbedeckung, die sich nach einer Anwachsphase (oder Einheilung) im Laufe der Zeit nicht weiter vergrößert. Dadurch stellt diese Schicht eine überraschend geringe Diffusionsbarriere dar, wobei ebenfalls überraschend ist, dass ein stationärer Zustand auch im Sinne einer Diffusion von Analyten erreicht wird.

Erfindungsgemäß bevorzugt ist, dass die Erkennungsmarker über Adsorption, kovalente Bindung oder Linker an den implantierbaren Artikel gebunden sind.

Durch die geeignete Bindungsmethode ist es dem Fachmann möglich, dass faktische Einwachsverhalten des (gewünschten) Zelltyps zu steuern.

Für Absorption geeignete Artikeloberflächen sind dabei bevorzugt ausgewählt aus der Gruppe bestehend aus Titan, med. Edelstahl wie bevorzugt 316L, CoCr, Magnesium und Polymeren. Polymere können dabei sowohl unter Einsatzbedingungen im Körper abbaubar als auch am Körper permanent sein.

Für kovalente Bindungen geeignete Gruppen auf der Oberfläche des implantierbaren Artikels sind bevorzugt ausgewählt aus der Gruppe bestehend aus Hydroxyrest, Aminorest Carbonylrest und Mercaptogruppe.

Ein Linker im Sinne der vorliegenden Erfindung ist ein Molekülteil, das die Verbindung zwischen dem spezifischen Erkennungsmarker für Zellen und der Oberfläche des implantierbaren theranostischen Artikels chemisch gewährleistet. Der Linker ist aufgebaut aus einer Ankergruppe und einer Spacergruppe. Die Spacergruppe weist dabei eine Kettenlänge von 1-30 bevorzugt 5-12 Atomen auf.

Geeignete bevorzugte Ankergruppen sind:
Acylsäure, Phosphonate, Thiole, Isocyanate und besonders bevorzugt Isothiocyanate.
Als Spacer finden bevorzugt Verwendung:
PEG, Polyprolin, Adipinsäure, bevorzugt Aminohexansäure.

Weitere Reagenzien zur Kopplung wie N,N'-Carbonyldiimidazol (CDI), 1-(3-Dimethylaminopropyl)-3-ethylcarbodiimid (EDC) oder Disulfosuccinimidyl-tatrat (DST) sind unter bestimmten Einsatzbedingungen ebenfalls bevorzugt.

Bevorzugt im Sinne der Erfindung ist, dass der spezifische Erkennungsmarker ein Oligopeptid ist. Oligopeptide umfassen bis zu zehn Aminosäuren und können sowohl aufgrund ihrer Größe als auch auf Basis ihrer Funktionsweise als Erkennungsmarker für bestimmte Zelltypen besonders gut im Sinne der vorliegenden Erfindung eingesetzt werden.

Ganz besonders bevorzugt im Rahmen dieser Erfindung ist ein erfindungsgemäß implantierbarer Artikel, wobei der molekulare Erkennungsmarker eine RGD- oder cRGD-Sequenz umfasst oder aus ihr besteht.

Besonders bevorzugt sind im Rahmen der vorliegenden Erfindung ist der spezifische Erkennungsmarker für Zellen, ausgewählt aus der Gruppe bestehend aus Verbindungen der Formel I 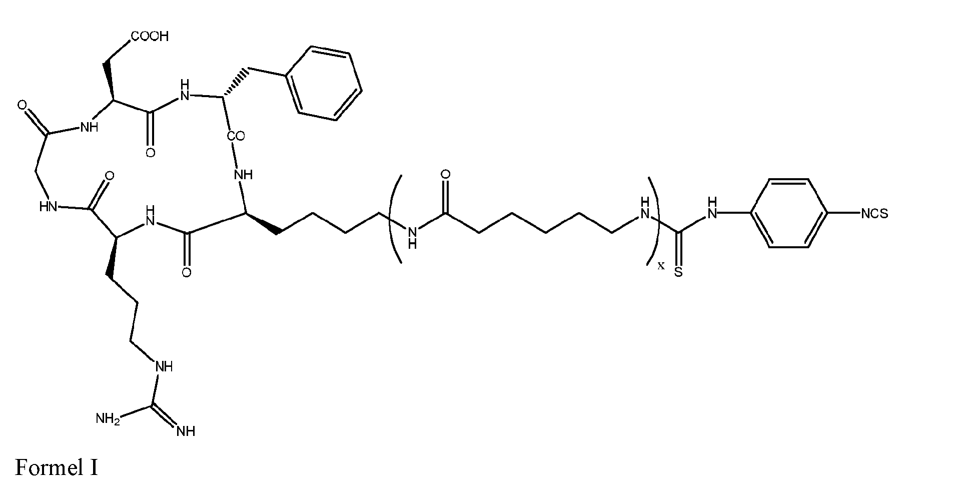 x = 0, 1, 2, 3, 4, 5 oder 6
und
Verbindungen der Formel II 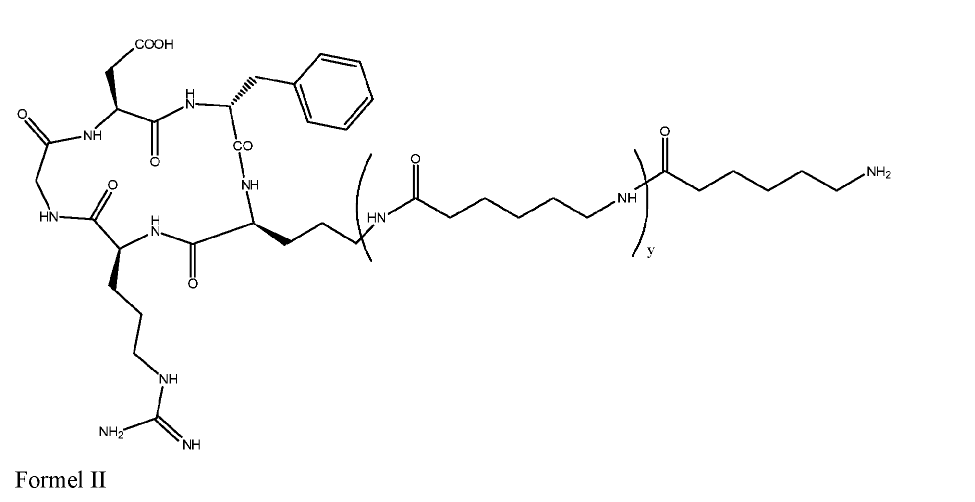 y = 0, 1, 2, 3, 4 oder 5.

Erfindungsgemäß bevorzugt ist ferner, dass die Oberfläche des Artikels metallischen, keramischen oder Polymercharakter besitzt.

Zusätzlich zu den erfindungsgemäß vorzusehenden spezifischen Erkennungsmarkern für Zellen kann die Oberfläche des implantierbaren theranostischen Artikels bevorzugt gegen die Anlagerung von Störfaktoren aus dem Körper, insbesondere aus der Körperflüssigkeit, sofern der implantierbare Artikel mit Körperflüssigkeit in Verbindung kommt, passiviert werden. Dies kann z. B. mittels geeigneter Polymere oder Hydrogele wie z. B. auf Basis von Polyethylenglykol (PEG) erfolgen.

Erfindungsgemäß bevorzugt kann die Oberfläche des implantierbaren theranostischen Artikels weitere Modifikationen tragen, die spezielle Bestandteile der Körperflüssigkeit binden oder abweisen.

Als solche Modifikationen können anorganische oder organische Moleküle über physikalische Absorption oder kovalente Bindung an die Artikeloberfläche geknüpft werden, wie z. B. Polymere, Peptide, Proteine, Aptamere, molekular-geprägte Polymere, RNA, DNA, PNA, LNA, siRNA und Nanopartikel. Die Oberfläche der erfindungsgemäßen Artikel kann eine Nano- oder Mikrostrukturierung tragen. Es können zu Strukturierung auf der Oberfläche sowohl runde sphärische, sphärische, zylindrische, kegelförmige, quadratische, rechteckige, wie auch längliche Strukturen aufgetragen wie auch abgetragen werden. Dazu zählen Rillen, Röhren, Vollzylinder, Kugeln, Halbkugeln, Quader und Würfel.

Erfindungsgemäß bevorzugt ist, dass der implantierbare theranostische Artikel ein Wirkstoffdosierungssystem umfasst. Mit Wirkstoffdosierungssystem ist hier ein implantierbarer Artikel gemeint, die über einen längeren Zeitraum kontrolliert ein Medikament absondern kann. Erfindungsgemäße Closed-Loop Systeme kombinieren ein erfindungsgemäßes Sensorsystem, das bestimmte Parameter in Körperflüssigkeiten misst, wie z.B. Glukose, mit einem Wirkstoffdosierungssystem, wie z.B. zur Abgabe von Insulin, so dass kritische physiologische Werte zeitnah korrigiert werden können oder deren Auftreten verhindert werden.

Für diesen implantierbaren theranostischen Artikel eignet sich die erfindungsgemäße O-berflächenmodifikation besonders gut, da viele dieser Artikel regelmäßig mit Körperflüssigkeit, insbesondere Blut in Kontakt kommen, wo ein definiertes Einwachsen besonders hohe Bedeutung besitzt.

Besonders überraschend in diesem Zusammenhang ist - wie oben bereits angedeutet -dass für die erfindungsgemäßen implantierbaren theranostischen Artikel ein Sensor mit der erfindungsgemäß vorzusehenden Oberflächenbelegung durch spezifische Erkennungsmarker für Zellen erreicht werden kann, dass die entstehende Zellschicht in einem ausreichenden Maße durchlässig für die jeweiligen Analyten ist. Dies gilt sogar für den Fall, dass der Teil der Oberfläche des implantierbaren Sensors, der durch eine semipermeable Membran (durchlässig für den gewünschten Analyten) gebildet wird, ebenfalls an seiner (Außen-) Oberfläche spezifische Erkennungsmarker für Zellen im Sinne dieser Erfindung umfasst.

Bevorzugt umfasst der erfindungsgemäße theranostische Artikel einen Sensor wie oben beschrieben, wobei der Sensor ein Sensor für einen molekularen Bestandteil einer Körperflüssigkeit ist.

Ein molekularer Bestandteil im Sinne dieses Textes ist ein Bestandteil des Körpers, der vom Körper selbst gebildet und/oder von ihm aufgenommen wird, wie z.B. ein Elektrolyt sowie ein Molekül aus der Gruppe der Kohlehydrate, Metabolite, Stoffwechselprodukte, Peptide, Fette, Lipide, Proteine, Neurotransmitter, Polyelektrolyte, Nukleotide, Hormone, Nanopartikel oder Wirkstoffe, Aminosäuren, Fettsäuren, Desoxyribonukleinsäuren, Ribonukleinsäuren sowie Wasser.

Weiterhin bevorzugt ist ein erfindungsgemäßer Artikel, der ein Sensor für einen molekularen Blutbestandteil oder Bestandteil ist.

Besonders bevorzugt ist ein erfindungsgemäßer Sensor, der ein Glukosesensor ist.

Glukose als Analyt ist wirtschaftlich relevant, da im Rahmen von Diabeteserkrankungen eine möglichst Echtzeitkontrolle des jeweiligen Glukosespiegels im Blut erwünscht ist. Diabetes Mellitus ist eine weit verbreitete Krankheit mit mehreren Millionen Erkrankten allein in den USA.

Gleichzeitig ist Glukose als Analyt besonders geeignet, da es sich hierbei um ein verhältnismäßig kleines Molekül handelt und somit ein gutes Diffusionsverhalten gewährleistet ist.

Weiterhin bevorzugt sind Analyten gewählt aus der Gruppe bestehend aus Elektrolyten, Kohlehydraten, Metaboliten, Stoffwechselprodukten, Aminosäuren, Peptiden, Fetten, Fettsäuren, Lipiden, Proteinen, Neurotransmittern, Polyelektrolyten, Ribonukleinsäuren, Desoxyribonukleinsäuren, Nukleotiden, Hormonen, Nanopartikeln, Wirkstoffen sowie Wasser. Im Speziellen sind bevorzugt: Albumine/Globuline, Alkalische Phosphatase, Alpha-1-Globulin, Alpha-2-Globulin, Alpha-1-Antitrypsin, Alpha-1-Fetoprotein, Alpha-Amylasen, Alpha-Hydroxybutyrat-Dehydrogenase, Ammoniak, Antithrombin III, Bicarbonat, Bilirubin, Carbohydrate-Antigen 19-9, Carcino-embryonales Antigen, Chlorid, Cholesterin, Cholinesterase, Cobalamin/Vitamin B 12, Coeruloplasmin, C-reaktives Protein, Cystatin C, D-Dimere, Eisen, Erythropoetin, Erythrozyten, Ferritin, Fetuin A Fibrinogen, Folsäure/Vitamin B9, Freies Tetrajodthyronin (fT4), Freies Trijodthyronin (fT3), Gamma-Glutamyltransferase, Glukose, Glutamat-Dehydrogenase, Glutamat-Oxalazetat-Transaminase, Glutamat-Pyruvat-Transaminase, Glykohämoglobin, Hämatokrit, Hämoglobin, Haptoglobin, Harnsäure, Harnstoff, HDL-Cholesterin, Homozystein, Immunglobulin A, Immunglobulin E, Immunglobulin G, Immunglobulin M, INR, Kalium, Kalzium, Kreatinin, Kreatin-Kinase, Kupfer, Laktat, Laktat-Dehydrogenase, LDL-Cholesterin, Leukozyten, Lipase, Lipoprotein, Magnesium, mittlere korpusukuläre Hämoglobin-Konzentration, mittleres korpuskuläres Hämoglobin, mittleres korspuskuläres Volumen, Myoglobin" Natrium, NT-proBNPBNP, Osmolalität, Partielle Thromboplastinzeit, Phosphat, pH-Wert, Plasma-Thrombin-Gerinnungszeit, Prostataspezifisches Antigen, Quick-Wert, Retikulozyten, Rheumafaktor, Thrombozyten, Thyreoidea stimulierendes Hormon, Transferrin, Triglyzeride, Troponin T

Der Begriff "Wirkstoff" umfasst typische Medikamente oder auch Methabolite, die für die Behandlung von Erkrankungen gegeben werden, wie Muskarinrezeptor-Antagonisten, neuromuskulär blockierende Stoffe, Cholesterinesterase-Hemmstoffe, Adrenozeptor-Agonisten, indirekt wirkende Sympathomimetika, Methylxanthine, alpha-Adrenorezeptor-Antagonisten, Mutterkornalkaloide, beta-Adrenozeptor-Antagonisten, Inaktivierungshemmstoffe, Antisympathonika, 5-HT- Rezeptor-agonisten, Histaminrezeptor-Agonisten, Hiastaminrezeptor-Antagonisten, Analgetika, Lokalanästhetika, Sedativa, Antikonvulsiva, Konvulsiva, Muskelrelaxantien, Antiparkinsonmittel, Neuroleptika, Antidepressiva, Lithium, Tranquillantien, Immunsuppressiva, Antirheumatika, Antiarrhythmika, Antibiotika, ACE-Hemmer, Aldosteronrezeptor-antagonisten, Diuretika, Vasodilatatoren, positiv i-notrope Substanzen, antithrombotische/thrombolytische Substanzen, Laxantien, Antidiarrhoioka, Pharmake bei Adipositas, Uricostatika, Uricosurika, Lipidsenker, Antidiabetika, Anithypoglykämia, Hormone, Iodsalze, Threostatika, Eisen, Vitamine, Spurenelemente, Virostatika, Antimykotika, Antituberkulotika, Substanzen bei Tumorchemotherapie als Wirkstoffe, interessant.

Im Speziellen sind typische Medikamente oder auch Methabolite, die bei koronaren Herzerkrankungen und der Herzinsuffizienz gegeben werden, wie Diuretika, ACE-Hemmer (Ramipril, Captopril), beta-Blocker (Carvedilol), Angiotensin Rezeptor-Blocker (Valsartan), Aldosteron-Blocker (Eplerenone, Spironolacton), Statine (Atorvastatin) interessant.

Viele dieser Analyten können zur Charakterisierung des Gesundheitszustandes von Individuen in den Körperflüssigkeiten bestimmt werden, insbesondere bei chronischen Erkrankungen, wie z. B. Herzinsuffizienz, Niereninsuffizienz. Die meisten interessanten Moleküle sind dabei klein genug, um ein gutes Diffusionsverhalten durch eine Endothelzellschicht in das Sensorinnere zu gewährleisten.

Teil der Erfindung ist ferner die Verwendung von spezifischen Erkennungsmarkern für Zellen, wobei die Erkennungsmarker ausgewählt sind aus der Gruppe bestehend aus Antigenen, Peptiden, Proteinen, Antikörpern, Aptameren, molekular-geprägten Polymeren und Polynukleotiden mit n ≥ 1 Monomereinheiten (DNA, RNA, PNA, LNA). zur Verhinderung von Biofouling und/oder Thrombenbildung auf der Oberfläche eines Implantats.

Die erfindungsgemäße Verwendung führt zu den Vorteilen der erfindungsgemäßen Implantate: Es wird - wie bereits oben beschrieben -das Einwachsverhalten der Implantate im implantierten Zustand definiert gesteuert. Die erfindungsgemäß zu verwendenden Erkennungsmarker wirken durch ihre Bindung an die Oberfläche des Implantates als Attraktionspunkte für bestimmte (gewünschte) Zelltypen, die auf der Oberfläche immobilisiert werden und anschließend eine Proliferation zeigen, so dass eine homogene (dünne) Zellbedeckung des Implantates entsteht. Wie ebenfalls oben angedeutet wird hierdurch dem Körper eine natürliche Oberfläche präsentiert, so dass Abwehrreaktionen des Körpers, wie z. B. Entzündungsreaktionen abgemildert oder unterbunden werden, ebenso wie Thrombenbildung. Die minimale Zellbedeckung erreicht nach der Einwachsphase einen konstanten Zustand, das heißt sie vergrößert sich im Laufe der Zeit nicht mehr, und stellt eine nur geringe und vor allen Dingen konstante Diffusionsbarriere dar. Dementsprechend kann ein Analyt - für den Fall, dass das theranostische Implantat ein Sensor ist-verhältnismäßig leicht in den Sensor gelangen und die Signale des Sensors sind über einen langen Zeitraum bei gleicher Analytenkonzentration konstant und somit reproduzierbar. Durch den stationären Zustand der Implantatoberfläche wird eine hohe Langzeitstabilität des Implantates ermöglicht.

Bestandteil der Erfindung ist auch ein Verfahren zum Herstellen eines erfindungsgemäßen implantierbaren theranostischen Artikels mit einer Biofouling vermindernden oder Biofoulling verhindernden Oberfläche und/oder eine Oberfläche zum Vermindern oder Vermindern von Thrombenbildung, umfassen die Schritte
a) Bereitstellen eines implantierbaren Artikels und
b) Versehen von Teilen der Oberfläche oder der Oberfläche des Artikels mit spezifischen Erkennungsmarkern für Zellen, wobei die Erkennungsmarker ausgewählt sind aus der Gruppe bestehend aus Antigenen, Peptiden, Antikörpern, Aptameren, molekular-geprägten Polymeren und Polynukleotiden mit n ≥ 1 Monomereinheiten (RNA, DNA, PNA, LNA).

Hierbei ist für den Fachmann selbstverständlich, dass durch geeignete weitere Schritte auch erfindungsgemäße implantierbare Artikel in den oben beschriebenen bevorzugten Ausführungsformen hergestellt werden können. Der Vorteil der erfindungsgemäßen Artikel bzw. der durch das erfindungsgemäße Verfahren hergestellten Artikel liegt darin, dass sie in vielen Bereichen des Körpers, also nicht nur z. B. subkutan, sondern auch im Kontakt mit der Blutbahn (z. B. intravasal) eingesetzt werden können. Hierzu ist lediglich eine geeignete Auswahl der spezifischen Erkennungsmarker sowie eine geeignete Aufbringung dieser Erkennungsmarker auf die Artikeloberfläche notwendig. Da die Auswahl der Marker den Zelltyp festlegt, der sich bevorzugt an der Oberfläche des erfindungsgemäßen Artikels anlagert, ist somit das Einwachsverhalten steuerbar. Selbstverständlich wird der Fachmann den spezifischen Erkennungsmarker für Zellen auch in Abhängigkeit vom gewünschten Einsatzort und natürlich in Abhängigkeit von der Einsatzbestimmung des Implantates, z. B. in einem Menschen oder einem bestimmten Tier, wählen.

Nachfolgend wird die Erfindung anhand von Beispielen weiter erläutert: Der Glukosesensor in den folgenden Beispielen beruht auf dem Prinzip der traditionellen amperometrischen Enzymsensoren mit immobilisierter Glucose-Oxidase: Hierin wird die Glukose selektiv durch die enzymatische Umwandlung von Glukose gemessen. Das Enzym Glukose-Oxidase ist in der Sensorspitze mit Hilfe eines Polymers immobilisiert und mit Glutaraldehyd vernetzt. Gemessen wird entweder die Abnahme von Sauerstoff oder die Bildung von Wasserstoffperoxid durch elektrochemische Reaktion der Glukose mit der Oxidase. Es findet entweder eine Oxidation an der Elektrode oder eine Reduktion an der Gegenelektrode statt.

Bevorzugt weist der implantierbare theranostische Artikel zusätzlich eine Telemetrieeinheit (Transceiver) auf, durch welche die ermittelten Werte der Analytenkonzentration an ein externes Gerät unidirektional oder bidirektional übertragen werden können. Darüber hinaus ist es sinnvoll, dass diese Werte auf diesem exteren Gerät angezeigt werden. Es ist weiterhin möglich, dass der implantierbare theranostische Artikel ein Wirkstoffdosierungssystem enthält, das in Abhängigkeit von der ermittelten Analytenkonzentration einen Wirkstoff, wie z.B. Insulin automatisch abgibt ("Closed-Loop"). Es ist weiterhin möglich, dass vom externen Gerät Daten an das Wirkstoffdosierungssystem übermittelt werden, die als Trigger für die Substanzabgabe fungieren. Diese Datenübertragung kann automatisch erfolgen. Alternativ ist es möglicht, dass manuell ein Trigger für das Wirkstoffdosierungssystem gesetzt werden kann.

Die in den Beispielen 1-3 beschichteten Artikel werden in eine große, herzferne Vene implantiert. Sie sollen zunächst nicht wandständig, frei im Blutstrom befestigt sein. Durch die spezielle Beschichtung werden Endothel-Progenitorzellen aus dem Blutstrom und Endothelzellen angelockt, die sich auf der Artikeloberfläche ansiedeln, proliferieren und nach einigen Tagen eine Monoschicht Endothel bilden. Die Endothelzellen wachsen auch über das semipermeable Sensorfenster, bilden aber genügen große Zwischenzell-Poren aus, dass der Analyt diffundieren kann.

Die Erfindung wird im Folgenden beispielhaft anhand eines Sensors beschrieben. Selbstverständlich können diese beispielhaften Ausführungsbeispiele auch auf andere theranostische Artikel angewandt werden, wobei es sich bei diesen Artikeln beispielhaft um Wirkstoffdosierungssysteme oder aktive Implantate wie Herzschrittmacher, Defibrillatoren, Kardioverter, Nervenstimulatoren handeln kann.

### Beispiel 1 für Oberflächenbeschichtung:

Ein im Sauerstoffplasma oder durch Spülen mit der Lösungsmittelreihe Dichlormethan, Aceton, Methanol und Millipore Wasser gereinigter Sensorkopf wird wie folgt weiterbehandelt:

Es wird eine 1 mM Lösung von Hydroxyundecylphosphonsäure in trockenem Tetrahydrofuran (THF) hergestellt. In diese wird der Sensorkopf gehängt und das Lösungsmittel wird innerhalb von einer Stunde abgedampft, wobei der Meniskus der Lösung über die Sensoroberfläche wandert.

Anschließend wird der Sensorkopf 18 Stunden bei 120°C getempert und daraufhin mit Lösungsmittel THF gespült.

Die so vorbehandelte Oberfläche wird 15 Stunden in eine 0,3 M Lösung von Carbonyldiimidazol (CDI) in trockenem Dioxan gelegt. Anschließend wird das Substrat zwei mal 10 Minuten mit trockenem Dioxan gespült und anschließend im Stickstoffstrom getrocknet.

Auf die so behandelte Oberfläche wird eine Lösung der zu koppelnden Verbindungen (hier ein cyclisches Pentapeptid gemäß Formel II mit y = 2 (ca. 50 µg/ml) in PBS-Puffer (aminosäurefrei) gegeben und über Nacht bei 4°C geschüttelt. Anschließend wird der Sensorkopf mit Puffer gespült.

### Beispiel 2 für Oberflächenbeschichtung:

Ein gemäß Beispiel 1 gereinigtes Sensorgehäuse aus Titan (Ti), das aus einem Zylinder mit Durchmesser 3-7 French besteht, das an einem Kopfende eine Durchführung für ein Sensorkabel und am anderen Ende ein Sensorfenster bestehend aus einer semipermeablen Membran besitzt, wird wie folgt weiterbehandelt:

Es wird eine 3 mM Lösung von 3-(4-Oxybenzophenone)propylphosphonsäure in trockenem Tetrahydrofuran hergestellt.

Mit dieser Lösung wird die gereinigte Oberfläche drei Mal besprüht. Anschließend wird das Gehäuse 12 Stunden bei 120°C getempert und daraufhin mit dem Lösungsmittel THF gespült.

Das Titangehäuse wird in eine Lösung der zu koppelnden Verbindungen (hier ein cyclisches Pentapeptid gemäß Formel II mit y = 2 (ca.500 µg/ml) in PBS-Puffer gemäß Beispiel 1 gegeben und über Nacht bei 4°C geschüttelt.

Am nächsten Tag werden die Ti-Sensoroberflächen aus dem Lösungsmittel entfernt, getrocknet und bei 260 nm mit 100 mW/cm² belichtet.

Nichtgebundenes Protein wird abgewaschen.

### Beispiel 3 für Oberflächenbeschichtung:

Die gereinigten Sensorgehäuse aus Titan (siehe Beispiel 2) werden in einer Mischung aus Toluol, Triethylamin und 3-Aminopropyltriethoxysilan gegeben und 14 Std. bei Raumtemperatur inkubiert. Nach Ablauf der Reaktion wird der Sensor in Toluol gewaschen und für 1 Std. bei 135 °C getempert.

Zusammensetzung der Silanisierungslösung:
10 ml Toluol, getrocknet
0,5 ml Trietylamin
1 ml Silan 3 Aminopropyltriethoxysilan

An den Reinigungsschritt (spülen des Ti Substrates mit Trichlormethan) schließt sich die Aktivierung mit 1,1'-Carbonyl Diimidazol (CDI) an.

Die silanisierten und gespülten Ti-Substrate werden für 5 Stunden in CDI gegeben. Dazu wird das CDI in trockenem Dioxan gelöst. Hier eignet sich eine Stammlösung von 2,5 g / 50 ml CDI in Dioxan, die für mehrere Tage (2, trocken) haltbar ist. Die Substrate werden bei Raumtemperatur leicht bewegt.

Nach der Aktivierung werden die Substrate entnommen und mit trockenem Dioxan gespült.

Für die Ankopplung der cyclischen Peptide gemäß Formel I mit x = 2 werden die aktivierten Ti-Substrate in die Peptid Lösung einer Konzentration von 5 mg/ml, getaucht und bei 4°C über Nacht (min. 12 Std.) gekoppelt.

Die Reaktion geschieht geeigneter Weise in 125 mM Natriumborat mit 0,066% SDS bei einem pH-Wert von 10.0.

Die Lösung ist danach wieder verwendbar bzw. es können mehrere Oberflächen mit dieser Lösung behandelt werden.

Die Sensoren werden nach der Kopplung dreimal mit 5 ml des Borax-Puffers (oben) gewaschen. Danach noch dreimal mit Wasser. Die nach diesen Waschschritten noch analysierbaren Peptide sind kovalent gebunden.

Die in den Beispielen 1 bis 3 beschichteten implantierbaren Sensoren zeigten allesamt nach Implantation und Einwachsphase eine definierte, einschichtige, zeitlich unveränderliche und nicht-trombogene Vernarbung. Die Sensoren waren allesamt für wenigstens 6 Monate einsatzfähig

Die semipermeablen Membranen der Sensoren blieben für den Analyten in konstanter Weise permeabel, so dass zuverlässige und reproduzierbare Signale erzeugt wurden. Dementsprechend haben erfindungsgemäße implantierbare Artikel allgemein eine Funktionsfähigkeit nach den Implantieren von wenigstens drei Monaten, bevorzugt wenigstens sechs Monaten und besonders bevorzugt wenigstens einem Jahr auf.

## Patentansprüche

1. Implantierbarer theranostischer Artikel, umfassend auf seiner Oberfläche spezifische molekulare Erkennungsmarker für Zellen, wobei die Erkennungsmarker ausgewählt sind aus der Gruppe bestehend aus Peptiden, Proteinen, Antikörpern, Antigenen, Aptameren, molekular-geprägten Polymeren und Polynukleotiden.

2. Implantierbarer theranostischer Artikel nach Anspruch 1, wobei die Erkennungsmarker über Adsorption, kovalente Bindung oder Linker an den implantierbaren Artikel gebunden sind.

3. Implantierbarer theranostischer Artikel nach Anspruch 1 oder 2, wobei der spezifische Erkennungsmarker ein Oligopeptid ist.

4. Implantierbarer theranostischer Artikel nach einem der vorangehenden Ansprüche, wobei der spezifische Erkennungsmarker eine RGD- oder cRGD-Sequenz umfasst oder aus ihr besteht.

5. Implantierbarer theranostischer Artikel nach einem der vorangehenden Ansprüche, wobei der Artikel zusätzlich eine Telemetrieeinheit aufweist, durch welche die ermittelten Werte der Analytenkonzentration an ein externes Gerät und Werte und Triggersignale vom externen Gerät zum Artikel übertragen werden können.

6. Implantierbarer Artikel nach einem der vorangehenden Ansprüche, wobei der Artikel einen Sensor für einen molekularen Bestandteil einer Körperflüssigkeit enthält.

7. Implantierbarer theranostischer Artikel nach Anspruch 6, wobei der Artikel einen Sensor für einen molekularen Blutbestandteil enthält.

8. Implantierbarer theranostischer Artikel nach einem der Ansprüche 6 oder 7, wobei der Sensor ein Glukosesensor ist.

9. Implantierbarer theranostischer Artikel nach einem der vorangehenden Ansprüche umfassend zusätzlich oder alternativ zum Sensor ein Wirkstoffdosierungssystem, das in Abhängigkeit von der ermittelten Analytenkonzentration einen Wirkstoff automatisch abgibt.

10. Verwendung von spezifische Erkennungsmarkern für Zellen, wobei die Erkennungsmarker ausgewählt sind aus der Gruppe bestehend aus Peptiden, Proteinen, Antikörpern, Antigenen, Aptameren, molekular-geprägten Polymeren und Polynukleotiden (RNA, DNA, PNA, LNA). zur Verminderung oder Verhinderung von Biofouling und/oder Thrombenbildung auf der Oberfläche eines implantierbaren theranostischen Artikels.

11. Verfahren zum Herstellen eines implantierbaren theranostischen Artikels mit einer Biofouling vermindernden oder Biofouling verhindernden Oberfläche und/oder einer Oberfläche zum Vermindern oder Verhindern von Thrombenbildung nach einem der Ansprüche 1 bis 6, umfassend die Schritte:
a) Bereitstellen eines implantierbaren Artikels und
b) Versehen von Teilen der Oberfläche oder der Oberfläche des Artikels mit spezifischen Erkennungsmarkern für Zellen, wobei die Erkennungsmarker ausgewählt sind aus der Gruppe bestehend aus Peptiden, Proteinen, Antikörpern, Antigenen, Aptameren, molekular-geprägten Polymeren und Polynukleotiden (RNA, DNA, PNA, LNA).
